Europäisches Patentamt

European Patent Office

Office européen des brevets

(19)

(11) EP 1 022 329 B1

(12) FASCICULE DE BREVET EUROPEEN

(45) Date de publication et mention
de la délivrance du brevet:
04.05.2005 Bulletin 2005/18

(51) Int Cl.⁷: **C12N 1/14**, C12N 9/34,
C12N 9/62, C12N 9/24,
C12P 7/06, A23K 1/165
// (C12N1/14, C12R1:685)

(21) Numéro de dépôt: 00400188.9

(22) Date de dépôt: 25.01.2000

(54) **Composition multienzymatique à activités glucoamylasique, protéolytique et xylanasique et procédé pour sa production par fermentation à l'état solide de son de blé avec Aspergillus**

MutienzymKomposition mit Glucoamylase, Protease und Xylanase Aktivität, und Verfahren zu deren Herstellung durch Aspergillus Gärung im festen Getreideweizenmedium

Multi-enzyme composition comprising glucoamylolytic, proteolytic and xylanolytic activities, and process to produce it by solid state fermentation of wheat bran by Aspergillus

(84) Etats contractants désignés:
AT BE CH CY DE DK ES FI FR GB GR IE IT LI LU MC NL PT SE

(30) Priorité: 25.01.1999 FR 9900775

(43) Date de publication de la demande:
26.07.2000 Bulletin 2000/30

(73) Titulaire: GIE Agro Industrie
Romilly sur Seine, 10100 Romilly sur Seine (FR)

(72) Inventeurs:
• Labeille, Pierre Jean
51100 Reims (FR)
• Baret, Jean-Luc Alain Guy
77250 Veneux les Sablons (FR)
• Duchiron, Francis Lucien
51100 Reims (FR)

(74) Mandataire: Colas, Jean-Pierre
Cabinet JP Colas
37, avenue Franklin D. Roosevelt
75008 Paris (FR)

(56) Documents cités:
• LABEILLE P ET AL: "Comparative study of wheat flour saccharification and ethanol production with two glucoamylase preparations." INDUSTRIAL CROPS AND PRODUCTS., vol. 6, no. 3-4, 1 août 1997 (1997-08-01), pages 291-295, XP002119202 ELSEVIER., NL ISSN: 0926-6690

• ABRAHAM T E ET AL: "Development of an alternate route for the hydrolysis of cassava flour." STARCH STARKE., vol. 41, no. 12, 1989, pages 472-476, XP002119203 WILEY-VCH VERLAG, WEINHEIM., DE ISSN: 0038-9056
• HAN M S ET AL: "Saccharification and ethanol fermentation from uncooked starch using Aspergillus niger koji." KOREAN JOURNAL OF FOOD SCIENCE AND TECHNOLOGY, vol. 17, no. 4, 1985, pages 258-264, XP002119204
• GOWTHAMAN, M. K. ET AL: "Gas concentration and temperature gradients in a packed bed solid-state fermentor." BIOTECHNOL. ADV. (1993), 11(3), 611-20, XP002119205
• DATABASE FSTA [Online] INTERNATIONAL FOOD INFORMATION SERVICE (IFIS), FRANFURT/MAIN, DE STACHOVICZ K J ET AL: "Preliminary selection of mould strains producing proteolytic enzymes in submerged cultures." Database accession no. 74-3-08-I0602 XP002119207 & PRACE INSTYTUTOW I LABORATORIOW BADAWCZYCH PRZEMYSLU SPOZYWCZEGO, vol. 23, no. 2, 1973, pages 311-320, Inst. Przemyslu Fermentacyjnego, Warsaw, Poland
• ATCC FILAMENTOUS FUNGI NINETEENTH EDITION, 1996, page 52 XP002119206
• VELDMAN A.; VAHL H.A.: 'Xylanase in broiler diets with differences in characteristics and content of wheat' BRITISH POULTRY SCIENCE vol. 35, pages 537 - 550

Remarques:
    Le dossier contient des informations techniques présentées postérieurement au dépôt de la demande et ne figurant pas dans le présent fascicule.

Remarques:
    Le dossier contient des informations techniques présentées postérieurement au dépôt de la demande et ne figurant pas dans le présent fascicule.

## Description

L'invention concerne une composition à activités glucoamylasique, protéolytique et xylanasique et procédé pour sa production par fermentation à l'état solide de son de blé avec *Aspergillus niger.*

Il est connu de produire de l'éthanol à partir d'amidon de maïs par un procédé enzymatique comprenant une étape de liquéfaction de l'amidon par une alpha-amylase visant à hydrolyser l'amidon en dextrines, puis une étape de saccharification par une glucoamylase (appelée aussi amyloglucosidase) visant à hydrolyser les dextrines en glucose, et enfin une étape de fermentation de ce dernier en éthanol.

La mise en oeuvre des enzymes alpha-amylase et glucoamylase est généralement satisfaisante lorsqu'on part de laits d'amidon relativement purs obtenus par broyage par voie humide du maïs, mais lorsqu'on désire substituer des amidons de blé ou des farines de blé à l'amidon de maïs, on n'obtient pas de résultats satisfaisants avec ces deux seules enzymes en raison de la présence d'hémicelluloses qui accroissent la viscosité des moûts de farine saccharifiés au point que cela crée un problème pour l'exécution du procédé. Il faut utiliser dans l'étape de saccharification des enzymes auxiliaires comme des cellulases et des hémicellulases pour réduire la viscosité et remédier à ce problème. Par ailleurs, il est souhaitable d'utiliser également des protéases au cours de la saccharification afin d'hydrolyser les protéines de la farine et enrichir ainsi le moût en azote soluble en prévision de l'étape ultérieure de fermentation alcoolique. L'apport en source azotée nécessaire à la croissance des levures traditionnellement effectué pendant cette fermentation peut ainsi être réduit.

Toutes ces enzymes sont individuellement disponibles dans le commerce sous forme purifiée, mais ont l'inconvénient d'être relativement onéreuses et, donc, de grever, par conséquent, le coût de la production de l'éthanol de blé. En outre, il faut formuler des compositions à partir des enzymes individuelles, ce qui complique le procédé.

L'article d'ABRAHAM et al "Development of an alternate route for the hydrolysis of cassava flour" Starch Starke, vol 41, n° 12, 1989, pages 472-476, décrit la préparation et l'utilisation de son de blé fermenté à l'état solide avec *Aspergillus niger* comme source d'amyloglucosidase pour l'hydrolyse de farine de manioc. Le son de blé est humidifié et traité thermiquement, puis fermenté à l'état solide pendant 1 à 2 jours. Le procédé décrit par ABRAHAM permet d'obtenir une activité glucoamylasique faible (110-224 IU/g).

LABEILLE P et al : "Comparative study of wheat flour saccharification and ethanol production with two glucoamylase preparations", Industrial crops and products, vol 6, n° 3-4, 1er Août 1997, pages 291-295, décrit une préparation à partir de souches d'*Aspergillus niger,* d'une composition dénommée AAI et présentant des activités glucoamylasique, protéolytique et hémicellulase, destinées à la saccharification de farine de blé. L'article de LABEILLE P et al ne précise pas le procédé de fabrication de la composition AAI.

Il existe donc un besoin pour une composition, bon marché, combinant des activités glucoamylasique, protéolytique et hémi-cellulasique afin de pouvoir produire de l'éthanol à partir de farines de blé à un coût réduit.

L'invention vise à satisfaire ce besoin.

La présente invention concerne une composition selon la revendication 1.

De préférence, l'activité glucoamylasique est d'au moins 1 500 UG par gramme de matière sèche et/ou l'activité xylanasique est d'au moins 400 UX par gramme de matière sèche.

De préférence également, l'activité protéolytique est d'au moins 400 UP par gramme de matière sèche.

L'invention concerne aussi un procédé selon la revendication 8.

De préférence, l'activité glucoamylasique est d'au moins 1 500 UG par gramme de matière sèche et/ou l'activité xylanasique est d'au moins 400 UX par gramme de matière sèche.

De préférence également, l'activité protéolytique est d'au moins 400 UP par gramme de matière sèche.

De préférence, la souche d'*Aspergillus niger* est choisie parmi la souche NRRL 3112, la souche ATCC 76061 et les souches obtenues à partir desdites souches par sélection ou mutation lorsqu'on recherche une activité glucoamylasique élevée. La souche ATCC 76061 est particulièrement préférée.

Lorsqu'on recherche une activité glucoamylasique élevée, le son de blé utilisé comme matière de départ doit être un son non désamidonné. A part cette restriction on peut utiliser un son quelconque. De préférence, toutefois, le son comporte une proportion significative (au moins 40% en poids) de particules inférieures à 1 mm.

On donne ci-après, à titre illustratif et non limitatif, les caractéristiques de deux sons convenables.

| Caractéristiques | Son A | Son B |
|---|---|---|
| Humidité (%) | 12,3 | 19,5 |
| Teneur en protéines (% MH*) | 13,8 | 14,8 |
| Teneur en amidon (% MH*) | 24,6 | 21,3 |
| Granulométrie | | |
| >1,25 mm | 53,9 | 0,7 |

(suite)

| Granulométrie | | |
|---|---|---|
| entre 1,0 et 1,25 mm | 8,1 | 1,3 |
| entre 0,5 et 1,0 mm | 33,3 | 68,2 |
| entre 0,25 et 0,5 mm | 3,7 | 24,6 |
| entre 0,16 et 0,25 mm | 0,3 | 2,6 |
| < 0,16 mm | 0,7 | 2,6 |
| %MH = % par rapport à la matière humide. | | |

Le son de blé doit être humidifié et traité thermiquement en vue de le pasteuriser ou le stériliser. Il est avantageux que le traitement thermique ne précède pas l'humidification, car on a observé des résultats de fermentation médiocres dans le cas où on traite thermiquement le son avant de l'humidifier. Le traitement thermique peut consister en un chauffage par exemple dans un autoclave. Un traitement en autoclave de 20 mn à 120-121°C s'est avéré très satisfaisant, mais des conditions moins sévères (pasteurisation à 105°C, pendant 15 mn dans une étuve) conviennent aussi. Il est possible également d'opérer le traitement thermique du son en y injectant de la vapeur d'eau, ce qui peut permettre d'opérer simultanément l'humidification du son.

Avantageusement, on peut régler le pH lors de l'humidification dans la gamme de 4 à 5,5 afin d'améliorer l'effet pasteurisant du traitement thermique et le démarrage de la fermentation souhaitée.

Outre sa fonction de stérilisation, le traitement thermique a pour effet de favoriser la gélatinisation de l'amidon contenu dans le son de blé et, donc la disponibilité de ce substrat pour le champignon *Aspergillus niger,* ce qui permet des fermentations plus efficaces.

L'humidification du son est importante car la teneur en eau influe sur la performance de la fermentation. La teneur en eau initiale du son est réglée initialement à 50-60%, de préférence 50-55%, de la masse totale du son et de l'eau et on la maintient sensiblement dans cet intervalle pendant la fermentation, par exemple en procédant périodiquement à des apports d'eau pour compenser la perte en eau du milieu. L'expression "sensiblement maintenue" veut dire qu'il est tolérable que le taux d'humidité prenne une valeur s'écartant un peu ($\pm$ 5 unités %) de l'intervalle de 50-60% pendant une relativement brève période entre deux ajustements successifs du taux d'humidité ou en fin de fermentation. Il est avantageux, en tout cas, de ne pas descendre en dessous d'un taux d'humidité de 45%. Le taux d'humidité du milieu de culture tend à baisser au cours de la culture par évaporation sous l'effet de l'augmentation de température générée par la croissance fongique, ledit milieu étant un mauvais conducteur de la chaleur. La qualité de l'eau utilisée joue aussi un rôle non négligeable. On peut utiliser une eau courante de bonne qualité ou de l'eau distillée.

L'inoculation du son de blé peut se pratiquer avec tout inoculum approprié. L'homme du métier connaît de multiples façons de préparer un inoculum convenable à partir d'une souche sélectionnée. La dose d'inoculation est avantageusement d'au moins 1 x 10⁷ spores/gramme de matière sèche initiale.

La fermentation peut être conduite dans tout réacteur approprié. Des exemples de réacteur utilisable sont ceux décrits dans l'article de A. DURAND et Coll. publié dans Agro-Food-Industry Hi-Tech (Mai-Juin 1997, pages 39-42).

La fermentation peut être conduite pendant une période de 1 à 3 jours, de préférence de 30 à 60 heures. En dessous de 1 jour, la fermentation est par trop incomplète. Au bout de 3 jours la fermentation est achevée ou quasiment achevée de sorte qu'il serait non économique de la prolonger davantage. La température du milieu est typiquement maintenue entre 28 et 38°C, de préférence entre 32 et 36°C, ce qui correspond au domaine d'activité optimum connu des souches d'*Aspergillus niger* à utiliser dans l'invention. Avantageusement, à cette fin, la température de l'air est réglée à 34-38°C pendant les premières heures de la fermentation pour favoriser la germination des spores, puis réduite à 28-32°C pour le reste de la fermentation pour contribuer à la régulation de la température du milieu.

Le pH du milieu de fermentation n'est pas régulé habituellement. Si sa valeur de départ est voisine de 6,0-6,4, le pH baisse à 3,8-4,2, au cours de la culture, puis remonte à la fin. Ce retour est généralement corrélé avec la phase de sporulation du champignon. Le suivi de l'évolution du pH constitue un bon indicateur de l'état de la culture.

Le fermenteur doit être aéré, de préférence en continu, afin d'apporter l'oxygène nécessaire à la fermentation et éviter l'accumulation excessive de dioxyde de carbone produit par la fermentation. En outre, l'aération participe au contrôle de la température et de l'humidité du milieu de culture. L'air sera, de préférence, sensiblement saturé en eau pour limiter la tendance à l'assèchement du milieu. Il est difficile de donner des indications quantitatives sur le débit d'aération, car de multiples variables, en particulier la taille et la géométrie du réacteur, la quantité du son chargée, etc...., interviennent. De simples essais de routine permettront, toutefois, à l'homme de l'art de déterminer aisément un débit d'aération convenable dans chaque cas pratique.

La charge de son dans le fermenteur doit être périodiquement ajoutée à l'aide des moyens d'agitation, tels que des bras agitateurs, des lames ou spatules, ou des vis sans fin au cours de la fermentation en vue d'éviter la formation de masses imperméables et afin que l'aération intéresse de la façon la plus homogène possible toute la masse de son. Il faut, toutefois, éviter une agitation trop vigoureuse qui pourrait nuire au champignon.

La composition de l'invention est une composition solide qui est utile notamment pour la production d'éthanol à partir du blé. Elle peut être directement ajoutée à l'amidon liquéfié (dextrines) obtenu dans l'étape de liquéfaction, afin de procéder à la saccharification. Pour cette application, c'est l'activité glucoamylasique qui est le facteur le plus important. On utilisera donc, de préférence une composition de l'invention ayant une activité glucoamylasique, par exemple d'au moins 750 UG, de préférence d'au moins 1500 UG par gramme de matière sèche.

Une autre utilisation possible de la composition de l'invention concerne la production d'aliments pour animaux monogastriques, par exemple la volaille et les porcs, à base de blé. Dans cette application, c'est l'activité xylanasique qui constitue le facteur le plus important. On utilisera donc, de préférence, dans cette application, une composition ayant une activité xylanasique élevée, par exemple d'au moins 400 UX par gramme de matière sèche.

La composition de l'invention peut être séchée ou congelée en vue de sa conservation, si désiré.

Le séchage doit être réalisé à une température modérée pour ne pas affecter l'activité enzymatique. Un chauffage en étuve à 40°C s'est révélé approprié, par exemple. La congélation, de son côté, peut être réalisée sur la composition humide à basse température, par exemple à -20°C.

Dans les exemples, les diverses activités enzymatiques ont été mesurées par les méthodes suivantes :

a) Activité glucoamylasique

L'action d'une préparation de glucoamylase (GA) sur une solution d'amidon entraîne la libération de sucres réducteurs. Chauffés à 100°C en présence d'acide 3,5-dinitrosalicylique (DNS), ces compositions prennent une couleur brune mesurée au spectrophotomètre (Kontron Instruments, Milan, Italie) à 540 nm.

Le milieu réactionnel contient

- Solution d'amidon 1 % :        500 μl
- Tampon citrate 0,1 à pH 4,5        450 μl
- Solution enzymatique :        50 μl

La réaction se déroule pendant 30 min. à 60°C (55°C pour les GA d'A.orizae). Des prélèvements sont réalisés toutes les 5 min., mélangés avec du DNS et placés dans un bain glacé. Ils sont ensuite chauffés 5 min. à 100°C, refroidis rapidement puis dosés à 540 nm.

Nous avons établi ces conditions de dosage après avoir étudié l'influence de la température et du pH sur l'activité de nos préparations de GA. De l'amidon soluble Merck (Darmstadt, Allemagne) a été employé comme substrat de cette hydrolyse enzymatique. Le DNS est préparé selon le protocole proposé par P. Bernfeld, Methods in enzymology, 1, 149-158 (1955) qui est le suivant :

⇒ Dissoudre préalablement :

* 10 g d'acide 3,5-dinitrosalicylique,
* 200 ml de soude 2 molaires
* 200 ml d'eau distillée.

⇒ Ajouter ensuite :

* 300 g de tartrate double de sodium potassium.

⇒ Compléter le volume à 1 litre avec de l'eau distillée après dissolution totale :

Une fois préparé, ce réactif doit être conservé à l'abri de la lumière. Les courbes d'étalonnage ont été établies avec du glucose comme produit de référence pour le dosage de l'activité glucoamylase ou pour le suivi des réactions de liquéfaction- saccharification, et avec du xylose pour mesurer l'activité xylanase.

Une unité d'activité glucoamylase (UG) correspond à la quantité d'enzyme nécessaire à la libération d'une micromole d'extrémités réductrices par minute dans les conditions du dosage avec le glucose comme référence. L'activité glucoamylase, calculée à l'aide de la formule indiquée ci-dessous, est rapportée à la quantité de matière sèche intiale (MSI):

$$A = (P/Venz)*(Vferm/Mferm)$$

* A correspond à l'activité GA exprimée en $UG.gMSI^{-1}$ ($\mu mol.min^{-1}.gMSI^{-1}$),
* P correspond à la vitesse de libération d'équivalents glucose en $\mu mol.min^{-1}$,
* Venz représente le volume de la solution d'enzymes dosée en ml,
* Vferm est le volume total d'eau distillée utilisé pour extraire la solution d'enzymes en ml,
* Mferm, exprimée en g de MSI, correspond à la masse initiale de produit sec dont a été extraite la solution d'enzymes.

b) Activité protéase

Ce dosage a été mis au point sur de l'azocaséine selon la méthode de Béinon, décrite dans l'ouvrage "Proteins Purification Methods - a Practical Approach", Harris E.L.V. et Angal, S (Editeurs), IRL-Press, Oxford University Press, 1-66 (1989). La dégradation de ce substrat par des protéases entraîne la libération de groupements azo qui absorbent dans l'UV à 340 nm. L'évolution de l'absorbance pendant la cinétique d'hydrolyse de cette protéine indique l'importance de la réaction.

Le milieu réactionnel contient :

* Solution d'azocaséine à 1 %, pH 5,0 :       1000 $\mu$l
* Solution enzymatique :       200 $\mu$l

L'azocaséine (Sigma, Saint-Louis, Etats-Unis) est dissoute dans un tampon acétate 0,1 M à pH 5,0. On a dosé les activités protéases à ce pH car l'azocaséine est insoluble dans le tampon acétate à des pH inférieurs. La réaction enzymatique est réalisée à 60°C. Des prélèvements sont réalisés toutes les 5 min. pendant 20 min. et mélangés avec de l'acide trichloroacétique (TCA) à 5 % pour stopper la réaction.

Une unité d'activité protéase (UP) correspond à la quantité d'enzymes nécessaire à l'augmentation de 0,01 unité $A_{340nm}$ par minute, générée par la libération de groupes azo dans les conditions citées précédemment. Cette activité, calculée d'après la formule indiquée ci-dessous, est rapportée à la matière sèche initiale ($UP.G^{-1}$ MSI) ou l'activité glucoamylase ($UP.UG^{-1}$) :

$$A = (P/Venz)*(Vferm/Mferm)$$

* A correspond à l'activité protéase exprimée en $UP.gMSI^{-1}$,
* P correspond à la vitesse de libération des groupements azo exprimée en augmentation de 0,01 unité $A_{340nm}.min^{-1}$,
* Venz représente le volume de la solution d'enzymes dosée en ml,
* Vferm est le volume total d'eau distillée utilisé pour extraire la solution d'enzymes en ml,
* Mferm, exprimée en g de MSI, correspond à la masse initiale de produit sec dont a été extraite la solution d'enzymes.

c) Activité xylanase

Pour mettre en évidence cette activité enzymatique, on a fait agir les préparations de GA sur une solution de xylane soluble et on a mesuré les sucres réducteurs libérés par la méthode au DNS.

Le milieu réactionnel est composé de :

* Solution de xylane à 1 %, pH 4,5 :       900 $\mu$l
* Solution enzymatique :       100 $\mu$l

La solution de xylane de mélèze (Sigma à 1 % est préparée dans du tampon citrate à pH 4,5 et la réaction se déroule à 60°C. Des prélèvements sont effectués toutes les 5 min. pendant 20 min., mélangés avec du DNS et placé dans un bain glacé. Ils sont ensuite dosés selon un protocole identique à celui exposé pour la mesure des activités GA avec le xylose comme référence.

Une unité d'activité xylanase (UX) correspond à la quantité d'enzymes nécessaire à la libération d'une micromole de sucres réducteurs par minute. Cette activité est rapportée à la matière sèche initiale ($UX.g^{-1}$ MSI) ou à l'activité glucoamylase ($UX.UG^{-1}$). Pour calculer cette activité, nous avons repris la formule définie pour le caclul des activités GA dans laquelle :

* A correspond à l'activité xylanase exprimée en UX.gMSI$^{-1}$ (µmol.min.$^{-1}$.gMSI$^{-1}$),
* P correspond à la vitesse de libération d'équivalents xylose en µmol.min$^{-1}$,
* Les autres termes de la formule ne sont pas modifiés.

Les exemples non limitatifs suivants sont donnés en vue d'illustrer l'invention

EXEMPLE 1 - Sélection de souches d'*Aspergillus*

On a étudié de façon comparative l'aptitude de sept souches différentes d'*Aspergillus* disponibles dans le commerce à produire de la glucoamylase par fermentation en milieu solide de son de blé.

Les essais ont été effectués sur 50 g de milieu de fermentation dans une fiole d'Erlenmeyer. Le milieu était constitué de 21,5 g de son de blé, de 27,5 g d'eau, et de 1 g d'amidon de blé. Le pH initial du milieu était de 6,0-6,5. Le milieu a été stérilisé pendant 20 min dans un autoclave à 120°C.

On a inoculé chaque milieu avec 2.10$^7$ spores de la souche à tester par gramme de matière sèche initiale. L'âge des spores était de 3 jours. On a laissé la fermentation se dérouler pendant 40 ou 50 heures, les fioles d'Erlenmeyer étant placées dans une étuve à 35°C. A la fin de la fermentation on a mélangé le milieu fermenté avec 150 ml d'eau distillée afin de mettre les enzymes produites en solution, puis on a filtré pour récupérer la solution enzymatique. On a centrifugé la solution pour éliminer les spores et particules résiduelles, puis on a conditionné la solution en flacons de 100 ml que l'on a conservée à -20°C jusqu'à l'analyse de l'activité glucoamylasique.

Les souches testées et les résultats obtenus sont récapitulés dans le Tableau 1 suivant :

| Réf. souches de collections | Durée de la FMS (h) | Ac. GA (UG.g-1 MSI) |
|---|---|---|
| *A. niger* ATCC 76060 | 50 | 627 |
| *A. niger* ATCC 76061 | 50 | 943 |
| *A. niger* MUCL 28815 | 40 | 710 |
| *A. niger* MUCL 28816 | 40 | 631 |
| *A. niger* NRRL 3112 | 50 | 1056 |
| *A. oryzae* ATCC 22788 | 50 | 903 |
| *A. oryzae* ATCC 42149 | 50 | 861 |

On voit que les souches *A. niger* NRRL 3112, *A. niger* ATCC 76061 et *A. oryzae* ATCC 226788 ont les meilleures activités en terme de production de glucoamylase.

Une autre propriété importante à prendre en considération, toutefois, est la stabilité de la glucoamylase produite. On a donc procédé à des essais de thermostabilité en effectuant des traitements thermiques des solutions enzymatiques à 55 et 60°C pendant 30 min et en mesurant l'activité de la glucoamylase au bout de ce temps. Ces traitements se rapprochent des conditions d'utilisation pour la saccharification de l'amidon. Il a été trouvé que les souches *A. niger* ATCC 76061 et *A. niger* NRRL 3112 donnaient les glucoamylases les plus stables (100% d'activité résiduelle après 30 min à 55°C et environ 50% d'activité résiduelle après 30 min à 60°C) alors que les souches *A. oryzae* ATCC 22788 et ATCC 42149 donnaient des glucoamylases ayant 0% d'activité résiduelle après 30 min à 60°C et 46% d'activité résiduelle après 30 min à 55°C. Ceci nous a donc conduit à sélectionner les souches ATCC 76061 et NRRL 3112 de *A. niger.* Par ailleurs la souche *A. niger* NRRL 3112 s'est avérée être génétiquement assez instable (perte d'activité après quelques cycles reproductifs) de sorte que la souche la plus préférée est la souche ATCC 76061 de *A. niger.* C'est cette souche qui a donc été utilisée dans les exemples suivants .

EXEMPLE 2 - Production de glucoamylases en cuves pilotes fournies par l'INRA de 50 l non stériles : importance du prétraitement du son de blé.

Les essais ont été réalisés avec un fermenteur non-stérile de 50 l conforme à celui décrit dans l'article de A. DURAND et collaborateurs précité (Figure 1) et du son de blé BCE (fourni par la distillerie Brie Champagne Ethanol, Provins, France). Deux modes de préparation du son ont été mis en oeuvre pour obtenir 5 kg milieu de culture à 55% d'humidité :

• son sec : le son est autoclavé 1 h à 105°C puis mélangé à de l'eau (essai F4C3) ;
• son humide : le son est humidifié à 45% dans un pétrin et autoclavé 20 min. à 121°C (essai F4C4).

Dans les deux cas, on effectue une inoculation avec $2.10^7$ spores.g$^{-1}$ MS et on règle la teneur en eau des milieux à environ 55%. Ils sont ensuite fermentés sur 10 cm de hauteur de couche dans des cuves aérées. Au cours de ces cultures, le milieu est strié par intermittence à l'aide d'une spatule pour réduire sa température. Pendant la fermentation, l'atmosphère est renouvelée en permanence par un air conditionné dont la température, l'humidité et le débit sont tels qu'indiqués dans les Tableaux.

Les résultats sont présentés dans les Tableaux 2 (essai F4C3) et 3 (essai F4C4).

Ces données permettent de dégager plusieurs conclusions :

- L'humidification du son de blé, préalablement au traitement thermique est nécessaire à une production de glucoamylases efficace. Au-delà de la décontamination, le traitement thermique du son de blé favorise vraisemblablement la gélatinisation de l'amidon ;
- Le pH apparaît comme un bon indicateur qualitatif de l'évolution de la croissance et de la production de GA fongiques, sans pour autant permettre d'estimer la quantité de GA obtenue :

- Une légère agitation du milieu (striage) n'altère pas la production d'enzymes ;
- L'évolution de la teneur en eau pendant ces deux fermentations indique un assèchement considérable du milieu de culture qui pourrait être préjudiciable à la croissance fongique.

EXEMPLE 3 : Production de glucoamylases en fermenteur pilote fourni par la Société FUJIWARA : importance du maintien de l'humidité du milieu au cours de la fermentation.

Cet essai a été mis en oeuvre avec un fermenteur pilote vendu par la Société FUJIWARA, Okayama, Japon, et du son de blé BCE. Il diffère notamment du fermenteur utilisé dans l'exemple 2 par le diamètre de la cuve qui est de 0,66 m contre 0,35 m pour les cuves INRA. Dans ce fermenteur, 20 kg de milieu à 55% d'eau préparés selon le mode son humide décrit dans l'exemple 2 sont nécessaires pour mettre en oeuvre une culture sur 12 cm d'épaisseur. L'agitation est assurée par trois vis sans fin verticales à rotation constante qui viennent mixer le milieu dans la cuve en rotation (5-10 min/tour). Au cours de la fermentation, comme pour les cuves INRA de l'exemple 2, l'atmosphère gazeuse est renouvelée en permanence par un air conditionné dont la température, l'humidité et le débit sont tels qu'indiqués dans le Tableau 4.

Au cours de cet essai appelé FII, l'opportunité d'une régulation de l'humidité a été étudiée. Des mesures ponctuelles de l'humidité de la culture, effectuées avec un appareil à infrarouge, et de la masse de milieu sont utilisées pour déterminer la quantité d'eau à ajouter pour maintenir la teneur en eau du milieu au-dessus de 50%.

Les résultats de cet essai FII sont reportés dans le Tableau 4. Les résultats obtenus appellent les commentaires suivants :

- FII indique clairement que le maintien de la teneur en eau entre 50 et 55% favorise la production d'enzymes avec 1600 UG/g MS libérées après 44 h de fermentation, soit plus du double de l'activité obtenue au cours de l'essai F4C2 de l'exemple 2 qui n'avait pas bénéficié de cette régulation ;
- la stabilisation de la production d'enzymes dès 44 h de culture corrélée à l'apparition de spores fongiques montre qu'il n'est pas nécessaire de poursuivre la culture au-delà de cette phase ;
- une régulation satisfaisante de la température du milieu autour de 35°C peut être obtenue par une bonne combinaison du conditionnement de l'air et de l'agitation du milieu ;
- la culture supporte sans dommages le brassage intermittent mis en oeuvre par le système d'agitation du fermenteur FUJIWARA.

EXEMPLE 4 : Production de glucoamylases en fermenteur pilote INRA agité de 50 l : utilité d'un prétraitement thermique du son à la vapeur et d'une culture en "conditions stériles".

Ce fermenteur pilote est semblable à celui présenté dans WO-A-94 18306 et sur la figure 4 de l'article de A. DURAND et Collaborateurs précité. Cet outil permet de traiter le son à la vapeur directement dans le fermenteur, mode de préparation préféré à l'échelle industrielle. La culture est également préparée, inoculée et réalisée en conditions stériles à l'exception des prélèvements, ce qui confère une semi-stérilité à cet essai et diffère des deux précédents exemples.

A) Conditions expérimentales

9 kg de son BCE sont introduits dans le fermenteur, pré-humidifiés par 1,5 l d'eau, puis stérilisés en place 20 minutes à 121°C, avec une agitation périodique de 5 secondes toutes les 5 minutes. Ce traitement permet d'atteindre un taux d'humidité de 46%, ajusté ensuite à 55% lors de l'inoculation.

Le son est inoculé par une préparation de type koji :

180 g de son BCE (55% d'humidité initiale) fermentés 4 jours à 35°C sont mélangés à 3 litres d'eau stérilisée de façon à obtenir une suspension de spores qui constitue l'inoculum.

Les conditions initiales de la fermentation sont les suivantes :

18,3 kg de culture à 55% d'humidité et un pH initial de 5,7 ;
40 cm de hauteur de couche ;
Débit d'aération : 314 l.min$^{-1}$ ;
$T_{air\ d'entrée}$ : 35°C;
Humidité relative de l'air à l'entrée : 95%.

B) Suivi de la fermentation

Outre la mesure du pH, de la température du milieu, du pourcentage de matière sèche et de la production de GA, l'évolution de la masse de la culture est enregistrée en continu sur le fermenteur de 50 l agité, tandis que, pour le réacteur non stérile, des pesées de la culture sont effectuées à 21 h et à 42 h de fermentation.

Ces mesures de la masse présentent un double intérêt :

Maintien de l'humidité au cours de la culture par estimation du pourcentage de MS.

Pendant la fermentation deux phénomènes contribuent à la diminution de la masse de la culture, il s'agit :

- de l'assèchement du milieu d'une part que l'on désire compenser par un apport d'eau,
- de la perte de matière sèche d'autre part, liée à la croissance du champignon.

Cette perte de matière sèche n'est pas négligeable, 20% de MS étant perdus en 40 h de culture, soit 0,5% de MS par heure si l'on fait l'approximation d'une perte linéaire.

A partir de là, connaissant à chaque instant la masse de la culture ($M_{(t)}$), il est possible d'en déduire le pourcentage de MS théorique au temps t, par la relation :

$$\% \text{ MS}_{\text{théorique (t)}} = \frac{\text{MSI (MSI. 0,5\%).t}}{M_{(t)}}$$

où MSI est la quantité de matière sèche initiale.

Lorsque ce % MS ainsi calculé dépasse 50%, une addition d'eau stérilisée est effectuée de façon à ramener ce pourcentage à 45%.

Expressions des résultats par gramme de MS initiale.

Les évolutions de la masse et celles du pourcentage de MS mesuré permettent de calculer la perte de matière sèche réelle ($P_{MS}$ exprimée en %) au cours de la culture. Ainsi, la quantité de GA exprimée jusque là en UG.g$^{-1}$ MS peut être exprimée en UG.g$^{-1}$ MS initiale grâce à la relation suivante :

$$(\text{UG.g}^{-1} \text{ MSI}) = (\text{UG.g}^{-1} \text{ MS}) \cdot (100 - P_{MS})/100$$

C) Résultats

Les Tableaux 5 et 6 récapitulent les conditions opératoires et les résultats obtenus en réacteur agité sur son vapeur.

Malgré l'aération de la culture par un air saturé en humidité, l'assèchement du milieu est tel qu'il a été nécessaire à deux reprises de réajuster sa teneur en eau quand elle descendait en dessous de 50% comme indiqué sur le Tableau 5.

La température du milieu a pu être maintenue à une valeur moyenne de 35°C grâce à l'abaissement de l'air d'entrée, mais surtout par une agitation intermittente.

Dans ces conditions de culture, la croissance du champignon, dont on suit l'évolution par la mesure du pH, est maintenue pendant 60 h et permet d'atteindre une production de 1436 UG.g$^{-1}$ MS en 44 h et de 1990 UG.g$^{-1}$ MS en 63 h. Ramenée à la MS initiale, la quantité de GA produite est respectivement de 1160 et 1540 UG.g$^{-1}$ MSI. Pour comparaison, nous avions obtenu dans le cadre de l'exemple 3 en 44 h de culture 1605 UG.g$^{-1}$ MS équivalentes à 1067 UG.g$^{-1}$ MSI. Cette information est intéressante car elle indique que la productivité de ces deux essais est identique, mais que les conditions expérimentales de l'exemple 4 ont permis de prolonger la production d'enzymes même avec 40 cm de hauteur de couche.

Le traitement du son à la vapeur puis sa fermentation dans le réacteur INRA de 50l agité prolonge donc la culture fongique et la production d'enzymes.

Ramenée à la MS initiale, la quantité de GA produite est de 1540 UG.g$^{-1}$ MSI.

Sur les mêmes échantillons, des dosages d'activités xylanases et protéases ont été effectués. Les résultats obtenus sont très satisfaisants avec un maximum, en moyenne, à 50 h de fermentation de

350 UX.g$^{-1}$ MSI pour les xylanases ;
400 UP.g$^{-1}$ MSI pour les protéases.

Grâce à l'enregistrement en continu de la masse, il a été possible de calculer la perte de matière sèche au cours de la culture. Elle est d'environ 23% au bout de 60 h de culture (à 2% près compte tenu de la précision des pesées).

EXEMPLE 5 : Utilisation des sons fermentés produits dans l'Exemple 4 pour l'hydrolyse de farines de blé

Une série de saccharifications avec les sons fermentés obtenus dans l'exemple 4 a été menée sur des farines de blé préalablement soumises à un traitement de liquéfaction enzymatique classique. La préparation de glucoamylases AMG 300L® commercialisée par la Société NOVO a servi de témoin. Ces essais ont été réalisés avec une farine de blé conventionnelle type 45. Les conditions opératoires sont récapitulées dans le Tableau 7 pour 750 g de moût.

TABLEAU 7

| Produit | AMG 300L® (Novo) | Son fermenté | Son fermenté séché |
|---|---|---|---|
| Référence | AMG 300 L | Ex. 4 | Ex. 4 |
| Présentation | Liquide | Son humide | Son sec |
| Mode conservation | à +5°C | à -20°C | à T ambiante |
| Farine | Commerciale type 45 | Commerciale type 45 | Commerciale type 45 |
| Quantité utilisée (g) | 300 | 300 | 300 |
| Mat. Sèche du milieu (%) | 35 | 35 | 35 |
| Cond. Liquéfaction | 1h/88°C/pH 6,1 | 1h/88°C/pH 6,2 | 1h/88°C pH 6,2 |
| Enzyme | 125µl Termamyl. 120L® | 125µl Termamyl. 120L® | 125µl Termamyl. 120L® |
| Cond. Saccharif. | 44h/58°C/pH 4,6 | 40h/58°C/pH 4,55 | 44h/60°C/pH 4,52 |
| Qt. Equiv. à 3500 UG | 205 µl | 4,3g | 2,1g |

Au cours de ces hydrolyses de farine de blé, nous avons prélevé trois échantillons de milieu à chaque fois. Les résultats de concentrations en sucres réducteurs (S.R.) à différents moments de la saccharification présentés dans le Tableau 8 sont la moyenne de ces trois prélèvements. Ces dosages, effectués selon la technique au DNS, ont été réalisés sur les surnageants des prélèvements centrifugés. On a également mesuré la viscosité finale des produits saccharifiés.

TABLEAU 8

| Mesures | AMG 300L® (Novo) | Son fermenté | Son fermenté séché |
|---|---|---|---|
| Conc. S.R. ((initiale (g/l) | 180,9±4,6 | 185,0±3,1 | 171,5±4,5 |
| Conc. S.R. finale (g/l) | 327,5±18,5 | 325,0±22,5 | 348,3±19,1 |
| Viscosité (mPa.s) | 6,80 | 2,82 | 2,80 |

Par ailleurs, on a constaté un accroissement de la teneur en azote soluble des moûts après saccharification dû à l'action protéolytique du son fermenté.

Ces résultats indiquent que les sons fermentés produits dans l'Exemple 4 sont capables d'hydrolyser la farine de blé avec la même efficacité qu'une préparation standard de GA, quel que soit leur mode de stockage.

L'hydrolyse de la farine avec du son fermenté entraîne également une réduction notable de la viscosité comparée à une préparation enzymatique conventionnelle.

Exemple 6

Cet exemple illustre la possibilité de produire une quantité considérable de xylanases et peu de glucoamylases avec la souche d'Aspergillus niger.

Cet essai a été mis en oeuvre dans le fermeteur pilote Fujiwara avec du son BCE et une souche d'A. niger Ref. ATCC 201202 connue pour sa capacité à produire des xylanases. Le fonctionnement du fermenteur pilote est détaillé dans l'Exemple 3. 20 kg de milieu à 55 % d'humidité, préparés comme dans l'exemple 2, sont mis en oeuvre dans cet exemple. Comme dans l'exemple 3, durant la fermentation, l'humidité du milieu a été maintenue supérieure à 50 % et la température du milieu régulée autour de 35°C.

Au final, la souche A. niger ATCC 201202 a produit, après 37 heures dans ces conditions de fermentation, un son fermenté ayant 727 UX/g MS et 162 UG/g MS.

Exemple 7 : Intérêt de l'incorporation de son fermenté selon l'invention dans un aliment avicole à base de blé destiné à des poulets de chair.

Il est connu que les hémicellulases des farines de blé sont partiellement solubles dans l'eau et augmentent la viscosité du contenu intestinal, réduisant ainsi la libération et l'absorption de nutriments.

L'article de A. VELDMAN AND H.A. VAHL : "Xylanase in broiler diets with differences in characteristics and content of wheat" Br Poult Sci 1994 sept; 35(4) Pages 537-550 décrit des expériences destinées à mesurer l'effet d'un ajout de xylanases pures sur les performances zootechniques de volailles nourries avec une alimentation à base de blé.

Il a été démontré que l'ajout d'hémicellulases engendre une dégradation des hémicellulases, permettant ainsi de réduire la viscosité du contenu intestinal et d'améliorer la performance zootechnique d'animaux monogastriques tels que des poulets de chair, nourris avec des aliments dont la céréale unique est le blé.

Une expérimentation a été menée sur 1 200 poulets de chair Ross pour montrer l'intérêt de l'utilisation du son fermenté porteur d'activité hémicellulase (xylanase), en comparaison d'un aliment sans enzyme et d'un aliment contenant une source de xylanase standard, le produit Avizyme. Des aliments avec ou sans enzyme ont été préparés de façon à nourrir 4 lots de 300 poussins. Leur composition est détaillée dans le Tableau 9. Les aliments de croissance (AL CR) ont été utilisés pour les 21 premiers jours d'élevage puis ont été remplacés par des aliments de finition (AL FI) pendant 18 jours.

TABLEAU 9

| Aliment | AL CR | AL FI |
|---|---|---|
| Humidité (%) | 10,6 | 11,4 |
| Protéines (%) | 21,3 | 19,1 |
| Mat. Grasses (%) | 6,1 | 6,4 |

L'aliment 1 n'a reçu aucune enzyme. Les aliments 2 et 3 ont été additionnés de 3 et 5 kg, respectivement, de son fermenté par tonne d'aliment. L'aliment 4 a été additionné de 0,6 kg d'Avizyme ® par tonne d'aliment.

Les résultats de ce test au bout de 39 jours d'élevage sont résumés dans le Tableau 10.

TABLEAU 10

| Aliment | 1 | 2 | 3 | 4 |
|---|---|---|---|---|
| Son fermenté selon l'invention (kg/tonne)[a] | - | 3,0 | 5,0 | - |
| AVIZYME Finfeed (kg/tonne)[b] | - | - | - | 0,6 |
| Activité xylanase (UX/kg Aliment) | - | 1 700 | 2 840 | 1 620 |

a. ce son fermenté présentait une activité glucoamylasique de 1000 UG/g de matière sèche, une activité protéolytique de 125 UP/g de matière sèche et une activité xylanasique de 600 UX/g de matière sèche ;

b. l'Avizyme ® est fourni par la société Finfeed, Finlande;

TABLEAU 10   (suite)

| Aliment | 1 | 2 | 3 | 4 |
|---|---|---|---|---|
| Indice consommation 39 jours [c] | 1,775 | 1,748 | 1,738 | 1,745 |
| Réduction Indice Conso (% Alim. 1) [d] | - | 1,52 | 2,08 | 1,69 |
| Mortalité (%) | 2,3 | 2,0 | 3,0 | 2,3 |

c. rapport poids d'aliment consommé/gain de poids ;

d. c'est la réduction en % du poids d'aliment consommé par rapport au poids d'aliment 1 (sans enzyme) consommé.

L'incorporation de son fermenté à un aliment avicole (3 ou 5 kg/tonne) a permis de réduire significativement l'indice de consommation. Dans les conditions de l'essai, l'utilisation d'une dose de son fermenté supérieure à 3 kg/tonne paraît sans intérêt pratique. Les améliorations observées sont comparables à celles obtenues avec le produit commercial de référence Avizyme (0,6 kg/tonne). L'utilisation du son fermenté présente, toutefois, l'avantage d'être moins coûteux que l'utilisation du produit enzymatique du commerce.

EP 1 022 329 B1

**TABLEAU 5** - Paramètres physico-chimiques de la FMS en fermenteur de 50 l agité sur son vapeur

| Tps Cult (h) | Air, T entrée (°C) | Air, Débit (l/min) | Air, % HR | T milieu moyenne | pH moyen | Masse totale | Traitement |
|---|---|---|---|---|---|---|---|
| 0 | 35,0 | 314,0 | 94,6 | | 5,70 | 18,30 | |
| 13 | 36,1 | 312,7 | 92,6 | 38,8 | 4,72 | 18,00 | |
| 13 (après agit) | | 471,0 | | 38,6 | 4,68 | | agitation |
| 16 | 29,8 | 448,6 | 94,6 | 32,4 | 4,60 | 17,50 | agitation |
| 18 | 33,2 | 466,5 | 77,2 | 31,0 | 4,37 | 17,10 | |
| 20,33 | 32,6 | 466,5 | 93,7 | 34,3 | 4,20 | 16,80 | |
| 23,66 | | | | 34,2 | 4,13 | 15,80 | |
| 26 | 29,8 | 467,5 | 94,6 | 32,9 | 4,17 | 15,10 | |
| 26 | 29,8 | 467,5 | 94,6 | 33,3 | 3,81 | 17,30 | agitation + 2,5 l d'eau |
| 36,75 | 29,0 | 467,5 | 94,6 | | | | agitation |
| 36,75 | 27,0 | 467,5 | 94,6 | 29,9 | 3,71 | 14,30 | |
| 40,58 | 27,0 | 467,5 | 94,6 | 31,0 | 4,20 | 13,10 | |
| 40,66 | 27,0 | 467,5 | 94,6 | 31,7 | 3,60 | 15,10 | agitation + 2,1 l d'eau |
| 44,58 | | 448,6 | | 33,6 | 3,71 | 14,10 | |
| 47,17 | | 467,5 | | 34,8 | 4,04 | 13,30 | |
| 50 | 27,0 | 303,3 | 94,6 | 32,0 | 4,09 | 12,80 | agitation |
| 63,33 | 28,7 | 303,0 | | 31,0 | 5,75 | 10,30 | |

**TABLEAU 6** : Synthèse des résultats de la FMS en fermenteur de 50 l agité sur son traité à la vapeur

| Tps Cult (h) | Teneur en eau (%) | Perte de MS (%) | Perte en MS % (lissée) | UG/g MS moyenne | UG/g MSI moyenne | UX/g MS | UX/g MSI | UP/g MS | UP/g MSI |
|---|---|---|---|---|---|---|---|---|---|
| 0 | 54,50 | -2,07 | | 0,00 | 0,00 | 0,0 | 0,0 | 0,0 | 0,0 |
| 13 | 57,52 | 7,78 | 0,4 | 92,69 | 85,48 | 16,1 | 16,0 | 131,0 | 130,49 |
| 16 | 53,72 | 2,11 | 1,0 | 170,53 | 166,92 | 19,2 | 19,0 | 196,2 | 194,15 |
| 18 | 55,23 | 7,21 | 1,8 | 129,25 | 222,01 | 27,9 | 27,4 | 103,9 | 102,02 |
| 20,33 | 51,90 | 1,91 | 3,1 | 429,36 | 421,18 | 35,0 | 33,9 | 193,7 | 187,63 |
| 23,66 | 50,88 | 5,57 | 5,9 | 551,93 | 521,21 | 51,0 | 48,0 | 283,4 | 266,81 |
| 26 | 49,06 | 6,15 | 8,2 | 651,45 | 611,36 | 75,4 | 69,2 | 296,5 | 272,14 |
| 26 | 57,76 | 10,57 | 8,2 | 548,42 | 490,44 | 54,1 | 49,7 | 262,1 | 240,57 |
| 36,75 | 49,27 | 10,75 | 18,4 | 1127,09 | 1005,88 | 203,,5 | 166,2 | 359,1 | 293,20 |
| 40,58 | 51,14 | 20,55 | 20,5 | | | | | | |
| 40,66 | 58,72 | 22,54 | 22,0 | 1278,58 | 990,35 | 227,3 | 180,7 | 347,9 | 276,52 |
| 44,58 | 54,10 | 19,21 | 22,6 | 1436,16 | 1160,27 | 408,3 | 318,6 | 367,1 | 286,45 |
| 47,17 | 54,01 | 23,07 | 23,2 | 1535,69 | 1181,43 | 511,5 | 395,7 | 496,8 | 384,30 |
| 50 | 54,66 | 26,67 | 24,4 | 1742,78 | 1277,91 | 429,8 | 330,1 | 628,2 | 482,48 |
| 63,33 | 44,69 | 22,62 | 22,6 | 1989,07 | 1539,07 | 410,3 | 310,1 | 478,3 | 361,5 |

EP 1 022 329 B1

TABLEAU 2

|  | Tps Cult (h) | Air, T entrée (°C) | Air, Débit (l/min) | Air, Débit (m/s) | Air, % HR | T milieu moyenne | pH moyen | % Humidité | UGA/g MS moyenne | Tps Cult. (h) |
|---|---|---|---|---|---|---|---|---|---|---|
| F4C3 | 0,0 | 35 | 174,4 | 8,0 | 96 | 25,0 |  |  |  | 0,0 |
| son sec | 3,0 | 35 | 174,4 | 8,0 | 95 | 33,8 | 6,33 |  |  | 3,0 |
|  | 11,0 | 32 | 174,4 | 8,0 | 94 | 34,6 | 5,13 | 54,53 | 20,56 | 11,0 |
|  | 14,0 | 30 | 174,4 | 8,0 | 95 | 35,4 | 4,38 | 54,19 | 71,13 | 14,0 |
| Striage (17h) | 16,0 | 30 | 174,4 | 8,0 | 94 | 33,3 | 4,36 | 52,17 | 123,12 | 16,0 |
| Striage (18h) | 18,0 | 28 | 174,4 | 8,0 | 92 | 37,4 | 4,51 | 51,31 | 85,78 | 18,0 |
|  | 20,0 | 28 | 174,4 | 8,0 | 96 | 31,8 | 3,79 | 49,54 | 132,81 | 20,0 |
|  | 22,0 | 28 | 174,4 | 8,0 | 95 | 34,7 | 4,12 | 45,17 | 188,0 | 22,0 |
|  | 25,0 | 28 | 174,4 | 8,0 |  | 37,6 | 4,34 | 45,13 | 181,03 | 25,0 |
|  | 28,0 | 28 | 174,4 | 8,0 | 94 | 32,6 | 4,63 | 36,47 | 129,58 | 28,0 |
|  | 31,0 | 28 | 174,4 | 8,0 |  | 32,8 | 5,05 | 30,68 | 198,10 | 31,0 |
|  | 40,0 | 28 | 174,4 | 8,0 | 95 | 31,9 | 5,69 | 23,96 | 246,86 | 40,0 |

## TABLEAU 3

| | Tps Cult (h) | Air, T entrée (°C) | Air, Débit (l/min) | Air, Débit (m/s) | Air, % HR | T milieu moyenne | pH moyen | % Humidité | UGA/g MS moyenne | Tps Cult. (h) |
|---|---|---|---|---|---|---|---|---|---|---|
| F4C4 | 0,0 | 35 | 174,4 | 8,0 | 96 | 25,0 | 5,96 | | | 0,0 |
| son humide | 3,0 | 35 | 174,4 | 8,0 | 95 | 33,8 | 6,12 | | | 3,0 |
| | 11,0 | 32 | 174,4 | 8,0 | 94 | 35,1 | 5,06 | 53,93 | 135,77 | 11,0 |
| Striage (14h) | 14,0 | 30 | 174,4 | 8,0 | 95 | 39,9 | 4,66 | 53,04 | 121,02 | 14,0 |
| | 16,0 | 30 | 174,4 | 8,0 | 94 | 33,1 | 4,59 | 51,53 | 182,96 | 16,0 |
| Striage (18h) | 18,0 | 28 | 174,4 | 8,0 | 92 | 38,0 | 4,48 | 50,80 | 327,41 | 18,0 |
| | 20,0 | 28 | 174,4 | 8,0 | 96 | 34,3 | 3,72 | 51,76 | 360,25 | 20,0 |
| | 22,0 | 28 | 174,4 | 8,0 | 95 | 36,9 | 3,70 | 47,65 | 618,00 | 22,0 |
| | 25,0 | 28 | 174,4 | 8,0 | | 36,3 | 4,15 | 41,68 | 658,05 | 25,0 |
| | 28,0 | 28 | 174,4 | 8,0 | 94 | 30,9 | 4,53 | 35,73 | 660,03 | 28,0 |
| | 31,0 | 28 | 174,4 | 8,0 | | 30,8 | 5,18 | 33,98 | 583,62 | 31,0 |
| | 40,0 | 28 | 174,4 | 8,0 | 95 | 30,2 | 4,88 | 30,02 | 702,74 | 40,0 |

EP 1 022 329 B1

## TABLEAU 4

| | Tps (h) | Vitesse de venti-lation tours/mn | Humidité air (%) | T°C entrée air | T°C milieu | Humidité milieu % | pH | UG/g MS | UG/g MSI | Tps (h) |
|---|---|---|---|---|---|---|---|---|---|---|
| Agit. | 0 | 15 | 99,0 | 35,0 | 25,0 | 54,0 | 6,36 | 4,3 | 4,3 | 0 |
| | 10 | 15 | 98,5 | 35,0 | 34,7 | 53,7 | 5,95 | 23,9 | | 10 |
| | 12 | | | 33,0 | | | | | | 12 |
| | 15 | 15 | 98,6 | 33,0 | 34,5 | 51,6 | 4,92 | 133,3 | | 15 |
| Agit. + 3 l eau | 18 | 15 | 98,8 | 33,0 | 37,3 | 49,7 | 4,18 | 304,8 | 273,1 | 18 |
| | 21 | 15 | 98,3 | 30,0 | 30,7 | 54,7 | 3,83 | 472,1 | | 21 |
| | 24 | 15 | 98,2 | 30,0 | 31,9 | 52,4 | 3,76 | 709,2 | | 24 |
| Agit. + 2 l eau | 28 | 15 | 97,9 | 30,0 | 33,4 | 46,6 | 4,08 | 1198,0 | | 28 |
| | 29 | 25 | 98,0 | 30,0 | | 51,6 | | 1054,0 | | 29 |
| Agit. + 6 l eau | 38 | 15 | 98,6 | 32,0 | 31,5 | 39,2 | 5,00 | 1469,7 | 976,1 | 38 |
| | 39 | 15 | 99,0 | 32,0 | | 60,6 | | 1338,0 | | 39 |
| | 42 | 15 | 99,2 | 32,0 | 33,9 | 59,7 | 5,28 | 1514,8 | | 42 |
| | 44 | 15 | 98,1 | 32,0 | 34,1 | 54,8 | 5,76 | 1605,1 | 1067,6 | 44 |
| | 46 | 15 | 96,8 | 32,0 | 33,7 | 52,6 | 6,33 | 1601,0 | | 46 |
| | 66 | 15 | 99,0 | 32,0 | 32,4 | 34,0 | 7,28 | 1594,4 | 1161,7 | 66 |

EP 1 022 329 B1

**Revendications**

1.  Une composition présentant des activités glucoamylasique, protéolytique et xylanasique, **caractérisée en ce qu'**elle comporte du son de blé fermenté avec une souche d'*Aspergillus* choisie parmi les souches ATCC 201202, ATCC 76060, ATCC 76061, MUCL 28815, MUCL 28816, NRRL 3112 ou avec une souche d'*Aspergillus oryzae* choisie parmi les souches ATCC 22788 et ATCC 42149, lesdites activités enzymatiques glucoamylasique, protéolytique et xylanasique étant présentes aux valeurs minimales suivantes :

    -   glucoamylasique : au moins 100 UG par gramme de matière sèche
    -   protéolytique : au moins 100 UP par gramme de matière sèche
    -   xylanasique : au moins 100 UX par gramme de matière sèche,

    avec la condition que l'activité glucoamylasique soit d'au moins 750 UG par gramme de matière sèche et/ou l'activité xylanasique soit d'au moins 300 UX par gramme de matière sèche.

2.  Composition selon la revendication 1, **caractérisée en ce qu'**elle présente une activité glucoamylasique d'au moins 750 UG/g de matière sèche.

3.  Composition selon la revendication 1, **caractérisée en ce qu'**elle présente une activité glucoamylasique d'au moins 1500 UG/g de matière sèche.

4.  Composition selon la revendication 1, **caractérisée en ce qu'**elle présente une activité xylanasique d'au moins 300 UX par gramme de matière sèche.

5.  Composition selon la revendication 1, **caractérisée en ce qu'**elle présente une activité xylanasique d'au moins 400 UX par gramme de matière sèche.

6.  Composition selon la revendication 1, **caractérisée en ce que** la souche d'*Aspergillus niger* est choisie parmi la souche NRRL 3112, la souche ATCC 76061.

7.  Composition selon la revendication 6, **caractérisée en ce que** la souche d'*Aspergillus niger* est la souche ATCC 76061.

8.  Un procédé de production de la composition définie à la revendication 1, le son étant sous la forme d'une couche d'au moins 10 cm d'épaisseur, **caractérisé en ce qu'**il comprend les étapes consistant à (a) prendre du son de blé ; (b) à humidifier puis traiter thermiquement ledit son de façon à le pasteuriser ou le stériliser ; (c) à inoculer le son de blé résultant par une souche d'*Aspergillus* choisie parmi les souches ATCC 201202, ATCC 76060, ATCC 76061, MUCL 28815, MUCL 28816, NRRL 3112 ou avec une souche d'*Aspergillus oryzae* choisie parmi les souches ATCC 22788 et ATCC 42149 ; (d) le faire fermenter à l'état solide dans un réacteur aéré et agité périodiquement pendant une période de 1 à 3 jours, à une température de 28-38°C, ledit son étant réglé à une teneur en humidité initiale de 50 à 60% en poids, maintenue pendant la durée de la fermentation, dans des conditions d'aération propres à éviter une accumulation de dioxyde de carbone nuisible à la fermentation dans le réacteur et une élévation de la température due à la fermentation au-delà de la gamme indiquée, jusqu'à ce que la composition de fermentation présente les valeurs minimales suivantes d'activités enzymatiques :

    -   glucoamylasique : au moins 100 UG par gramme de matière sèche
    -   protéolytique : au moins 100 UP par gramme de matière sèche
    -   xylanasique : au moins 100 UX par gramme de matière sèche.

    avec la condition que l'activité glucoamylasique soit d'au moins 750 UG par gramme de matière sèche et/ou l'activité xylanasique soit d'au moins 300 UX par gramme de matière sèche.

9.  Procédé selon la revendication 8, **caractérisé en ce que** la souche d'*Aspergillus niger* est choisie parmi la souche NRRL 3112, la souche ATCC 76061.

10. Procédé selon la revendication 9, **caractérisé en ce que** la souche d'*Aspergillus niger* est la souche ATCC 76061.

11. Procédé selon l'une quelconque des revendications 8 à 10 **caractérisé en ce qu'**une dose d'inoculation est d'au

moins 1*10$^7$ spores/gramme de matière sèche initiale.

12. Procédé selon l'une quelconque des revendications 8 à 11, **caractérisé en ce qu'**il comporte l'étape supplémentaire consistant à congeler ou à sécher la composition obtenue dans l'étape (d).

13. Utilisation d'une composition selon l'une quelconque des revendications 1-3 et 4-7 dans la production d'éthanol à partir du blé.

14. Aliment pour animaux monogastriques incorporant une composition selon l'une quelconque des revendications 1 et 4-7.


**Patentansprüche**

1. Verbindung, welche Glukoamylase-, Protease- und Xylanase-Aktivitäten aufweist, **dadurch gekennzeichnet, dass** sie Weizenkleie umfasst, die mit einem Stamm von *Aspergillus* fermentiert wird, der unter den Stämmen ATCC 201202, ATCC 76060, ATCC 76061, MUCL 28815, MUCL 28816, NRRL 3112 ausgewählt wird, oder mit einem Stamm von *Aspergillus oryzae*, der unter den Stämmen ATCC 22788 und ATCC 42149 ausgewählt wird, wobei die Glukoamylase-, Protease- und Xylanase-Enzymaktivitäten bei den nachfolgenden minimalen Werten vorhanden sind:

   - Glukoamylase:      mindestens 100 UG pro Gramm Trockensubstanz
   - Protease:      mindestens 100 UP pro Gramm Trockensubstanz
   - Xylanase:      mindestens 100 UX pro Gramm Trockensubstanz

   unter der Bedingung, dass die Glukoamylase—Aktivität mindestens 750 UG pro Gramm Trockensubstanz ausmacht und/oder die Xylanase-Aktivität mindestens 300 UX pro Gramm Trockensubstanz ausmacht.

2. Verbindung nach Anspruch 1, **dadurch gekennzeichnet, dass** sie eine Glukoamylase-Aktivität aufweist, die mindestens 750 UG/g Trockensubstanz ausmacht.

3. Verbindung nach Anspruch 1, **dadurch gekennzeichnet, dass** sie eine Glukoamylase-Aktivität aufweist, die mindestens 1500 UG/g Trockensubstanz ausmacht.

4. Verbindung nach Anspruch 1, **dadurch gekennzeichnet, dass** sie eine Xylanase-Aktivität aufweist, die mindestens 300 UX pro Gramm Trockensubstanz ausmacht.

5. Verbindung nach Anspruch 1, **dadurch gekennzeichnet, dass** sie eine Xylanase-Aktivität aufweist, die mindestens 400 UX pro Gramm Trockensubstanz ausmacht.

6. Verbindung nach Anspruch 1, **dadurch gekennzeichnet, dass** der Stamm von *Aspergillus niger* unter dem Stamm NRRL 3112 und dem Stamm ATCC 76061 ausgewählt wird.

7. Verbindung nach Anspruch 6, **dadurch gekennzeichnet, dass** der Stamm von *Aspergillus niger* aus dem Stamm ATCC 76061 besteht.

8. Herstellungsverfahren der in Anspruch 1 definierten Verbindung, wobei die Kleie in Gestalt einer mindestens 10 cm dicken Schicht vorhanden ist, **dadurch gekennzeichnet, dass** es die Schritte umfasst, die daraus bestehen, (a) Weizenkleie zu nehmen; (b) die Kleie anzufeuchten und sie anschließend mit Wärme zu behandeln, damit sie pasteurisiert oder sterilisiert wird; (c) die daraus hervorgehende Weizenkleie mit einem Stamm von *Aspergillus* zu inokulieren, der unter den Stämmen ATCC 201202, ATCC 76060, ATCC 76061, MUCL 28815, MUCL 28816, NRRL 3112 ausgewählt wird, oder mit einem Stamm von *Aspergillus oryzae*, der unter den Stämmen ATCC 22788 und ATCC 42149 ausgewählt wird; (d) sie im festen Zustand in einem Reaktor, der in regelmäßigen Abständen belüftet und gerührt wird, während eines Zeitraumes von 1 bis 3 Tagen bei einer Temperatur von 28 bis 38 °C zu fermentieren, wobei ein anfänglicher Feuchtigkeitsgehalt der Kleie auf 50 bis 60 Gew.-% eingeregelt wird, der während der Fermentierungsdauer unter Belüftungsbedingungen beibehalten wird, die geeignet sind, um eine Ansammlung von Kohlendioxyd, die für die Fermentierung in dem Reaktor schädlich ist, und um aufgrund der Fermentierung einen Anstieg der Temperatur über den angegebenen Bereich hinaus zu vermeiden, bis die Fermentierungsverbindung die nachfolgenden minimalen Werte von Enzymaktivitäten aufweist:

- Glukoamylase:     mindestens 100 UG pro Gramm Trockensubstanz
- Protease:     mindestens 100 UP pro Gramm Trockensubstanz
- Xylanase:     mindestens 100 UX pro Gramm Trockensubstanz

unter der Bedingung, dass die Glukoamylase—Aktivität mindestens 750 UG pro Gramm Trockensubstanz ausmacht und/oder die Xylanase-Aktivität mindestens 300 UX pro Gramm Trockensubstanz ausmacht.

9. Verfahren nach Anspruch 8, **dadurch gekennzeichnet, dass** der Stamm von *Aspergillus niger* unter dem Stamm NRRL 3112 und dem Stamm ATCC 76061 ausgewählt wird.

10. Verfahren nach Anspruch 9, **dadurch gekennzeichnet, dass** der Stamm von *Aspergillus niger* aus dem Stamm ATCC 76061 besteht.

11. Verfahren nach irgendeinem der Ansprüche 8 bis 10, **dadurch gekennzeichnet, dass** eine Inokulationsdosis aus mindestens $1 * 10^7$ Sporen/Gramm anfänglicher Trockensubstanz besteht.

12. Verfahren nach irgendeinem der Ansprüche 8 bis 11, **dadurch gekennzeichnet, dass** es den zusätzlichen Schritt aufweist, welcher daraus besteht, die in Schritt (d) erhaltene Verbindung zu gefrieren oder zu trocknen.

13. Verwendung einer Verbindung nach irgendeinem der Ansprüche 1 bis 3 und 4 bis 7 für die Herstellung von Ethanol aus Weizen.

14. Futtermittel für monogastrische Tiere, welchem eine Verbindung nach irgendeinem der Ansprüche 1 und 4 bis 7 beigemengt wird.

**Claims**

1. Composition exhibiting glucoamylase, proteolytic and xylanase activities, **characterized in that** it consists of wheat bran fermented with an *Aspergillus* strain chosen among the ATCC 201202, ATCC 76060, ATCC 76061, MUCL 28815, MUCL 28816, NRRL 3112 strains or with an *Aspergillus oryzae* strain chosen among the ATCC 22788, ATCC 42149 strains, said enzymatic glucoamylase, proteolytic and xylanase activities being present at the following minimum values:

- glucoamylase: at least 100 GU per gram of dry matter,
- proteolytic: at least 100 PU per gram of dry matter,
- xylanase: at least 100 XU per gram of dry matter,

provided that the glucoamylase activity is at least 750 GU per gram of dry matter and/or the xylanase activity is at least 300 XU per gram of dry matter.

2. Composition according to claim 1, **characterized in that** it exhibits a glucoamylase activity of at least 750 GU/g of dry matter.

3. Composition according to claim 1, **characterized in that** it exhibits a glucoamylase activity of at least 1 500 GU/g of dry matter.

4. Composition according to claim 1, **characterized in that** it exhibits a xylanase activity of at least 300 XU per gram of dry matter.

5. Product according to claim 1, **characterized in that** it exhibits a xylanase activity of at least 400 XU per gram of dry matter.

6. Composition according to claim 1, **characterized in that** the *Aspergillus niger* strain is chosen from the NRRL 3112 strain, the ATCC 76061 strain.

**7.** Composition according to claim 6, **characterized in that** the *Aspergillus niger* strain is the ATCC 76061 strain.

**8.** Method for producing the composition defined in claim 1, the bran being in the form of a layer at least 10 cm thick **characterized in that** it comprises the stages consisting in (a) taking wheat bran; (b) moistening and then heat-treating said bran so as to pasteurize it or sterilize it; (c) inoculating the resulting wheat bran with an *Aspergillus* strain chosen among the ATCC 201202, ATCC 76060, ATCC 76061, MUCL 28815, MUCL 28816, NRRL 3112 strains or with an *Aspergillus oryzae* strain chosen among the ATCC 22788, ATCC 42149 strains, (d), fermenting it in the solid state in a reactor which is aerated and stirred periodically for a period of 1 to 3 days, at a temperature of 28-38°C, said bran being adjusted to an initial moisture content of 50 to 60 wt.% which is substantially maintained during fermentation, under aeration conditions appropriate for avoiding accumulation of carbon dioxide, which is harmful to the fermentation in the reactor, and a rise in temperature due to fermentation above the recommended range, until the fermentation composition exhibits the following minimum enzyme activity values:

- glucoamylase: at least 100 GU per gram of dry matter,
- proteolytic: at least 100 PU per gram of dry matter,
- xylanase: at least 100 XU per gram of dry matter,

provided that the glucoamylase activity is at least 750 GU per gram of dry matter and/or the xylanase activity is at least 300 XU per gram of dry matter.

**9.** Method according to claim 8, **characterized in that** the *Aspergillus niger* strain is chosen from the NRRL 3112 strain, the ATCC 76061 strain.

**10.** Method according to claim 9, **characterized in that** the *Aspergillus niger* strain is the ATCC 76061 strain.

**11.** Method according to any of claims 8 to 10, **characterized in that** an inoculation dose is at least $1.10^7$ spores per gram of initial dry matter.

**12.** Method according to any of claims 8 to 11, **characterized in that** it comprises the additional step consisting in freezing or drying the composition obtained in stage d).

**13.** Use of a product according to any of claims 1-3 and 4-7 in the production of ethanol from wheat.

**14.** Monogastric animal feed according to any of claims 1 and 4-7.